(19) Europäisches Patentamt
European Patent Office
Office européen des brevets

(11) EP 0 776 212 B1

(12) EUROPÄISCHE PATENTSCHRIFT

(45) Veröffentlichungstag und Bekanntmachung des Hinweises auf die Patenterteilung:
**02.06.1999 Patentblatt 1999/22**

(51) Int. Cl.$^6$: **A61K 35/12**, C07K 1/34

(21) Anmeldenummer: 95928949.7

(22) Anmeldetag: **18.08.1995**

(86) Internationale Anmeldenummer:
**PCT/DE95/01094**

(87) Internationale Veröffentlichungsnummer:
**WO 96/05846** (29.02.1996 Gazette 1996/10)

(54) **VERFAHREN ZUR HERSTELLUNG VON INFEKTIONSFREIEN PHARMAZEUTISCHEN PRÄPARATEN UND/ODER NAHRUNGSMITTELN AUS INFEKTIÖSEM, INSBESONDERE PRIONEN ENTHALTENDEM, MATERIAL**

METHOD OF PRODUCING INFECTION-FREE PHARMACEUTICAL PREPARATIONS AND/OR FOODSTUFFS FROM INFECTIOUS MATERIAL, PARTICULAR MATERIAL CONTAINING PRIONS

PROCEDE DE PRODUCTION DE PREPARATIONS PHARMACEUTIQUES ET/OU DE PRODUITS ALIMENTAIRES EXEMPTS D'INFECTION A PARTIR D'UN MATERIAU INFECTIEUX CONTENANT NOTAMMENT DES PRIONS

(84) Benannte Vertragsstaaten:
AT BE CH DE DK ES FR GB GR IE IT LI LU MC NL PT SE

(30) Priorität: **19.08.1994 DE 4429558**

(43) Veröffentlichungstag der Anmeldung:
**04.06.1997 Patentblatt 1997/23**

(73) Patentinhaber:
**SANORELL PHARMA GmbH & CO.**
**D-72270 Baiersbronn (DE)**

(72) Erfinder: **NEBE, Carl, Thomas**
**D-68526 Ladenburg (DE)**

(74) Vertreter:
**Fritzsche, Thomas M., Dr. et al**
**Brienner Strasse 52**
**80333 München (DE)**

(56) Entgegenhaltungen:
| | |
|---|---|
| EP-A- 0 033 686 | WO-A-82/03568 |
| WO-A-83/00044 | DE-A- 3 219 248 |

**Beschreibung**

Die Erfindung betrifft ein Verfahren zur Herstellung von nicht-infektiösen pharmazeutischen Präparaten und/oder Lebensmitteln aus einem Ausgangsmaterial, das einen oder mehrere verschiedene Arten von infektiösen Erregern, insbesondere Prionen, enthält, bzw. bei dem nicht sicher ist, daß es frei von solchen Erregern ist.

Das wachsende Verständnis für den Stoffwechsel in Lebewesen hat dazu geführt, daß immer mehr Signal- und Transmittersubstanzen, wie beispielsweise Hormone, zur gezielten Steuerung und Beeinflussung von Körperfunktionen verwendet werden. Daher enthalten eine Vielzahl von pharmazeutischen Präparaten und Nahrungsergänzungsmitteln derartige Wirksubstanzen. Ein Großteil der hierfür eingesetzten Substanzen wird jedoch aus biologischen Quellen erhalten, d. h. durch Entnahme von Organen, Geweben oder Körperflüssigkeiten aus pflanzlichen, insbesondere jedoch aus tierischen oder gar menschlichen Spendern oder auch aus Zellkulturen davon.

Bei der Gewinnung von Substanzen aus lebenden Spendern besteht jedoch auch das Risiko, daß unwissentlich Material von Spendern verarbeitet wird, das mit einem oder sogar mit mehreren pathogenen Erregern infiziert bzw. verseucht ist. Dies stellt sowohl bei bekannten Erregern, wie beispielsweise dem Aids auslösenden HI-Virus, sowie beispielsweise bei den bislang unerkannten, beispielsweise den sogenannten Rinderwahnsinn (BSE) oder auch Scrapie auslösenden Erregern ein hohes Risiko bei der Herstellung solcher biologisch-therapeutischen Präparate dar. Biologisch-therapeutische Wirksubstanzen, wie beispielsweise das Immunsystem aktivierende und regulierende Wirkstoffe, werden industriell aus der Thymusdrüse von Kälbern, das menschliche Wachstumshormon hGH wird aus der Hypophyse und Heparin wird zum Beispiel aus dem Darm von Schweinen oder der Lunge von Rindern gewonnen. Weitere Substanzen werden aus verschiedenen Plazentaextrakten erhalten. Bei Substanzen, die aus Zellkulturen gewonnen werden, besteht ebenfalls ein Risiko, daß diese Viren oder Virusarten oder virusähnliche Partikeln, sog. virus-like-particles, oder Prionen enthalten oder damit kontaminiert sind.

Es ist daher bereits auf vielfältige Weise versucht worden, derart gewonnene Präparate garantiert infektionsfrei herzustellen. Eine hierzu häufig verwendete Methode ist die Hitzesterilisation, die jedoch bei vielen biologischen Therapeutika auch zu einer teilweisen oder sogar zu einer vollständigen Inaktivierung des biologischen Wirkstoffes führt. Im Falle des Erregers der spongiformen Enzephalopathie hat es sich sogar gezeigt, daß selbst Temperaturen von 130 °C keine hinreichende Sicherheit zu seiner Inaktivierung bieten. Auch mittels chromatographischen Verfahren, wie beispielsweise mit der sogenannten Gelausschlußchromatographie oder auch mittels Affinitätschromatographie, ist versucht worden, die Menge der vorliegenden Erreger zu reduzieren oder diese sogar vollständig zu entfernen. Eine weitere Methode zur Entfernung von unerwünschten pathogenen Keimen ist das vorzugsweise fraktionierte Ausfällen mittels Ammoniumsulfat.

Das am einfachsten durchzuführende und daher auch am häufigsten verwendete Verfahren ist die Ultrafiltration mittels Membranen, wobei die Erreger aufgrund ihrer größeren Volumenmasse von der Filtrationsmembran zurückgehalten werden, wohingegen der kleinere gewünschte Wirkstoff die Membran passieren kann. Die Porengröße solcher Ultrafiltrationsmembranen ist jedoch nicht konstant, sondern weist eine durch die Herstellung bedingte statistische Größenverteilung auf, so daß in jeder Membran zwangsläufig größere Poren vorliegen, durch die dann auch Bestandteile mit einem größeren Volumen bzw. Masse und damit auch Bakterien, Viren und Prionen bzw. sogenannte "langsame Viren" durch die Filtrationsmembran hindurchgelangen können, so daß regelmäßig im Filtrat noch eine u.U. infektiöse Menge des Erregers vorliegt.

Eine Titerreduktion von Erregern der spongioformen Enzephalopathie über 7 Zehnerpotenzen durch nur einen Verfahrensschritt ist bisher in der Literatur nur von Autoklaviervorgängen oder Alkalibehandlung her bekannt (Danner, K. (1991): Übertragung spongioformer Encephalopathien durch Arzneimittel, Pharm. Ind., 53, 614 - 623). Als maximal erreichbar gelten 8 Zehnerpotenzen durch 20 minütige Autoklavierung bei 133 °C oder einstündige Behandlung mit 1 N NaOH bei 20°C (Bundesgesundheitsamt: Bekanntmachung der Sicherheitsanforderungen an Arzneimittel aus Körperbestandteilen von Rind, Schaf und Ziege zur Vermeidung des Risikos einer Übertragung von BSE oder Scrapie vom 16. 2. 1994).

Alle zuvor genannten Verfahren haben gemeinsam, daß sie keine 100 %-ige Sicherheit dafür bieten, daß der potentiell vorliegende Erreger garantiert entfernt worden ist. Aus diesem Grunde schreiben die Arzneimittelzulassungsbehörden vor, daß bei der Herstellung von biologischen Therapeutika mehrere verschiedene der zuvor genannten Verfahren verwendet werden müssen, da mit nur einer Methode allein auch bei wiederholter Anwendung bislang keine 100 %-ige Inaktivierung des pathogenen Erregers sichergestellt war. Eine serielle Verwendung des gleichen Verfahrensschrittes wird daher von den Arzneimittelbehörden explizit als unerwünscht angesehen. Bei der "National Scientific Conference on Viral Safety and Evaluation of Viral Clearance from Biopharmaceutical Products" (Bethesda, Maryland, USA, 14. Juni 1995) lehnte beispielsweise Dr. J. Löwer vom Paul Ehrlich Institut (Arzneimittelsicherheitsbehörde) beim Vortrag von Dr. S. Manabe es aus diesen Sicherheitsgründen ab, infektiöses Material allein durch Ultrafiltration beispielsweise an mit Phagen validierten Membranen zu entfernen.

Aus M. Pocchiari et al., Archives of Virology, 1988, Seite 131 sowie aus der EP-A-0 307 373 ist es beispielsweise bekannt, bei der Herstellung von menschlichem Wachstumshormon aus der Hypophyse des Menschen zur Reduzierung der Scrapieinfektivität über einen Filter mit einem Ausschlußvolumen von 100 kD oder über einen Filter mit einer Porengröße von 25 nm zu filtrieren, wobei in beiden Fällen eine Abnahme der Infektivität des Infektionstiters um einen Faktor von 2,2 bzw. 2,0 Zehnerpotenzen erreicht wird. Diese Abreicherungsrate läßt sich gemäß dem dort beschriebenen Verfahren dadurch erhöhen, daß der Lösung soviel Harnstoff zugesetzt wird, daß sie eine Konzentration von 6 M aufweist. Mit Hilfe dieses Additives konnte der Titer des infektiösen Agens von Scrapie sowie des Creutzfeldt-Jakob-Syndroms um einen Faktor von 4,4 bzw. 5,6 Zehnerpotenzen verringert werden. Um sicherzustellen, daß das Endprodukt keine infektiösen Erreger mehr aufweist, werden jedoch von den Arzneimittelsicherheitsbehörden wesentlich höhere Entfernungsraten verlangt. G. Stiles, "Unconventional Viruses", A unique challenge in the manufacturing of biotherapeutics, Proceedings of the workshop on biopharmaceutical process validation, February 20, 1992, Basel, Schweiz, Microbiological Associates Inc. Rockville, Maryland, U.S.A. beschreibt, daß es durch eine Mischung von verschiedenen Aufreinigungsverfahren, wie beispielsweise Membranfiltration mit 6 molarem Harnstoff, Ausfällen mit Ammoniumsulfat, chromatographische Auftrennung, und insbesondere auch durch eine Auswahl der Spendertiere, (Verdünnung des infektiösen Materials) möglich ist, eine "Abreicherung" des Viruses auf über 22 Zehnerpotenzen zu erreichen.

Aus der WO 91/12027 ist ein Verfahren zur Bestimmung der Abreicherung von Viren in Lösungen und zur Bestimmung der Abreicherungsrate bekannt. Dabei wird das zu reinigende Material über eine Ultrafiltrationseinheit geleitet, deren Abreicherungsrate zuvor ermittelt wurde, indem der Filter oder die Filtrationseinheit mit Viren der Familie Leviviridae beaufschlagt und vor und nach der Filtration der Titer der Viren bestimmt und daraus die Abreicherungsrate ermittelt wurde.

Die Erfindung hat zum Ziel, ein Verfahren zur Herstellung von Biotherapeutika bzw. Lebensmitteln oder auch Additiven hierfür bereitzustellen, das auch im großtechnischen Maßstab einfach und bequem durchzuführen ist und das sicherstellt, daß bei der Verwendung der hiermit erhaltenen Produkte in pharmazeutischen Präparaten und/oder Nahrungsmitteln keine Infektion mehr auftreten kann, wenn dabei mit Prionen infiziertes oder sogar hochgradig infektiöses Ausgangsmaterial verwendet wird.

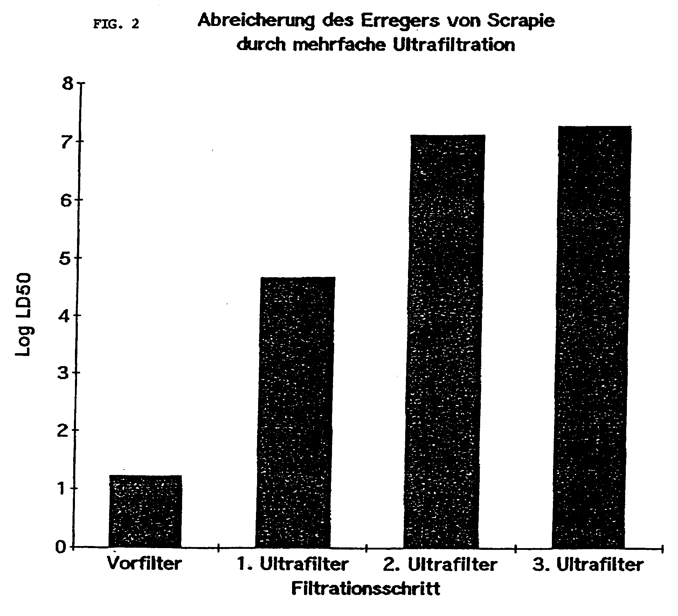

Dieses Ziel wird nun erfindungsgemäß dadurch erreicht, daß man ein gegebenenfalls mit infektiösen Prionen verseuchtes Ausgangsmaterial dadurch von den infektiösen Bestandteilen gänzlich befreit oder zumindest soweit befreit, daß es nicht mehr infektiös ist, indem man es wiederholt über eine Ultrafiltrationsmembran filtriert. Obwohl dies nicht zu erwarten war, wurde nämlich entgegen dem theoretisch zu erwartenden Ergebnis gefunden, daß bei einer wiederholten Filtration über die gleiche (oder auch eine andere) Membran die jeweilige Abreicherungsrate des infektiösen Agens ansteigt. Auf diese Weise ist es daher möglich, die Abreicherung des gegebenenfalls vorliegenden pathogenen Erregers durch eine mehrfache Wiederholung der Ultrafiltration beschleunigt zu entfernen und man kann daher auf andere Verfahren verzichten, d. h. es ist erfindungsgemäß möglich, den oder die Erreger bzw. Prionen lediglich durch die wiederholte Anwendung der Filtration zu entfernen. Wie von anderen Filtrationen bekannt ist, wäre zu erwarten gewesen, daß große Partikel zunächst abgereichert werden und die kleineren Partikel in nachfolgenden Filtrationen in immer geringer werdenden Abreicherungsraten entfernt werden können. Gerade dies ist jedoch, wie erfindungsgemäß gefunden wurde, bei Prionen nicht der Fall. Vielmehr werden diese bei der zweiten und bei nachfolgenden Filtrationen in immer höheren Raten entfernt (s. Fig. 2). Die einzelne Abreicherung nimmt daher mit dem 2. Filtrationsschritt zu und nicht ab.

Das erfindungsgemäße Verfahren hat sich als besonders geeignet für die Entfernung von sogenannten "langsamen Viren", also Prionen, erwiesen. Besonders bevorzugt wird das erfindungsgemäße Verfahren zum Entfernen des Erregers der spongiformen Enzephalopathie, insbesondere von Scrapie, Rinderwahnsinnn (BSE), Jakob-Creutzfeldt, Kuru, Gerstmann-Sträussler und Alzheimer, verwendet.

Ein weiterer Vorteil des erfindungsgemäßen Verfahrens liegt darin, daß gleichzeitig andere infektiöse Materialien wie Viren und Bakterien, sowie Endotoxin ebenfalls entfernt werden, was z. B. bei anderen Inaktivierungsverfahren nicht immer der Fall ist.

Bevorzugte Organe für die Gewinnung von Ausgangsmaterial sind Thymus, insbesondere Kalbsthymus, die Hypophyse von Tieren und Menschen, tierische und menschliche Plazenta sowie die Intestinalorgane vom Schwein und von Wiederkäuern, wie Rind, Ziege und Schaf. Auch Körperflüssigkeiten, insbesondere Blut, sind bevorzugt. Auch Biomoleküle aus Zellkulturen, wie z. B. Interleukin-2 aus stimulierten menschlichen Lymphozyten, Gewebs-Plasminogen-Aktivator (t-PA) aus Säugetierzellkultur oder rekombinante Glykoproteine sind nämlich ebenfalls potentiell mit pathogenen Erregern kontaminiert. Das erfindungsgemäße Verfahren ist auch besonders für die Gewinnung von Substanzen und Präparaten aus transgenen Tieren oder aus transgenen Zellkulturen geeignet, da diese häufig Zoonosen aufweisen.

Für das erfindungsgemäße Verfahren werden vorzugsweise Ultrafiltrationsmembranen verwendet, die einen Porendurchmesser von kleiner 100 nm, vorzugsweise von 50 - 0,5 nm, 30 -1 nm aufweisen, wobei 10 - 1 nm bevorzugt sind. Bevorzugte durchschnittliche Porengrößen lassen Moleküle mit einem Molekulargewicht von 30 kD die Membran nicht mehr passieren. Besonders bevorzugt sind Spiralmembranen und insbesondere Membranen mit Tangentialfluß, wie sie beispielsweise unter der Bezeichnung Amicon Spiralmembranen, z. B. S1Y30, S10Y30 und S100Y30, im Handel erhältlich sind. Besonders geeignet sind hydrophile Membranen, insbesondere mit geringen Adsorptionseigenschaften, wie Membranen aus regenerierter Cellulose, aus Polysulfon bzw. Gemischen davon.

Es hat sich als vorteilhaft erwiesen, die im erfindungsgemäßen Verfahren zu verwendende Ultrafiltrationsmembran vor ihrer Verwendung mit dem zu filtrierenden Ausgangsmaterial vorzubehandeln. Dazu wird über die hydrierte, vorgewässerte und zweckmäßigerweise mit einer physiologischen Kochsalzlösung getränkte Membran ein garantiert erregerfreies Ausgangsmaterial filtriert.

Es hat sich gezeigt, daß sich mit dem erfindungsgemäßen Verfahren allein mit der zweiten Ultrafiltration der Erreger um mindestens einen Faktor von $10^{-6}$, insbesonders um mindestens $10^{-7}$, abreichern läßt. Zweckmäßigerweise wird das zu reinigende Material so oft über eine Ultrafiltrationsmembran filtriert, bis sich im Filtrat keine Infektiosität mehr nachweisen läßt. Dies ist nach mindestens zweifacher, vorzugsweise drei- oder mehrfacher Ultrafiltration der Fall.

Das erfindungsgemäße Verfahren hat sich als besonders geeignet erwiesen, wenn das zu reinigende Material über einen Ultrafilter oder eine Ultrafiltrationseinheit geleitet wird, deren Abreicherungsrate zuvor ermittelt wurde, wie dies in der WO 91/12027 beschrieben ist. Dabei wird der Filter oder die Filtrationseinheit mit Viren der Familie Leviviridae beaufschlagt und vor und nach der Filtration der Titer der Viren bestimmt. Aus der Differenz der Viruskonzentration wird dann die Abreicherungsrate ermittelt. Bevorzugte, hierfür verwendbare Testviren sind die Leviviridae-Viren MS2, F2 oder R17. Als besonders zweckmäßig hat sich jedoch der Bakteriophage fr mit der Hinterlegungsnummer ATCC Nr. 15767-B1 erwiesen, wie dies in der zuvorgenannten WO 91/12027 näher beschrieben ist.

In einer weiteren bevorzugten Ausführungsform wird das erfindungsgemäße Verfahren mittels eines Druckhaltetestes kontrolliert, wie es in der WO 93/02714 beschrieben ist.

Bevorzugte mit dem erfindungsgemäßen Verfahren hergestellte Produkte sind die das Immunsystem stimulierenden bzw. regulierenden Substanzen des Thymus, sowie Heparin und menschliches Wachstumshormon, sowie über Säugerzellkulturen gewonnene natürliche oder rekombinante Moleküle.

Die Erfindung soll durch die folgenden Beispiele näher erläutert werden.

Beispiel 1

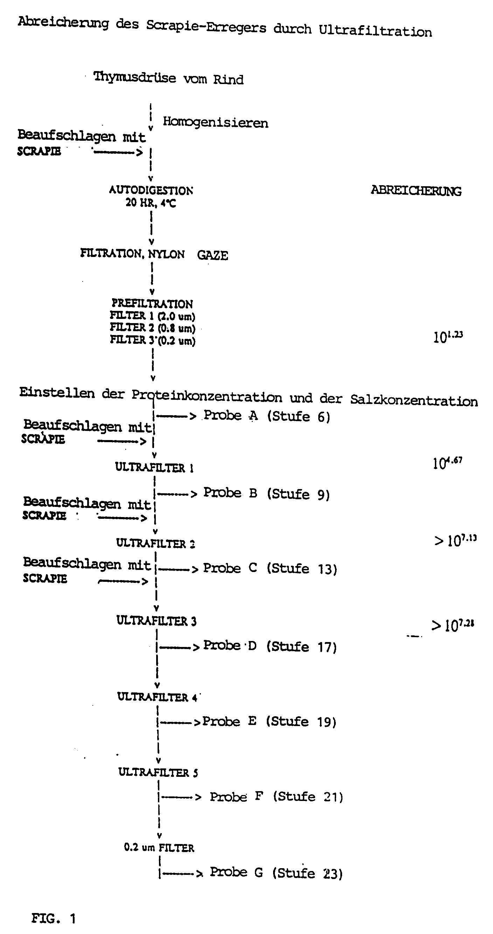

Vollständige Entfernung von Scrapie-Erregern Modellhaft für sog. langsame Viren bzw. Prionen wurden Scrapie-Erreger in einem Hamsterstamm vermehrt und nach Beaufschlagen auf einer produktionstypischen Ultrafiltrationsanlage von 5 seriellen Patronen abgereichert und die Infektiösität der Ultrafiltrate im Tierversuch ermittelt (siehe Fig. 1).

1.1 Gewinnung von infektiösen Scrapie-Erregern

Ein mit dem Scrapiestamm 263K infiziertes Gehirn eines Hamsters im Terminalstadium wurde maceriert und gepoolt. Davon wurden 500 mg in 4,5 ml einer sterilen physiologischen Salzlösung gegeben, homogenisiert und 10 Minuten lang bei 500 g zentrifugiert. 50 µl des Überstandes wurden intracerebral in 40 syrische LVG-Hamster injiziert. Die Tiere wurden bei Auftreten der Erkrankung mittels Genickbruch getötet, sobald dies aufgrund ihres klinischen Zustands notwendig war. Dies war üblicherweise 65 bis 80 Tage nach der Injektion der Fall. Die Gehirne wurden aseptisch entnommen, gefroren, maceriert, mit einer sterilen Kochsalzlösung versetzt und homogenisiert. Danach wurde 10 Minuten lang bei 500 g abzentrifugiert und der Überstand als "Spike"-Material verwendet.

1.2 Gewinnung von Thymusdrüsen-Homogenisat

Es wurden drei Thymusdrüsen von Kälbern püriert (ca. 550 g) und mit 1,5 l pyrogenfreiem Wasser versetzt und homogenisiert. Das Homogenisat wurde bei 4 °C bis zum Gebrauch gelagert.

Die Vorfilter und Ultrafilter wurden wie üblich mit Wasser gewaschen. Für die Vorfiltration wurde Thymusextrakt hintereinander über Nylongaze und drei Mikrofiltrationsmembranen (Pall, Nylonmembranen) mit Porengrößen von 2,0 µm, 0,8 µm und 0,2 µm filtriert. Um sicherzustellen, daß die Entfernung des Erregers nicht durch eine Absorption der Partikel an der großen inneren Oberfläche der spiralgewickelten Patronen verfälscht wird und um möglichst die alleinige Filtrationsabreicherung zu erfassen, wurden die für die Virusabreichueng einzusetzenden Filter vorbeschichtet. Dazu wurden sowohl die Vorfilter als auch die Filter mit dem gewonnenen Thymushomogenisat vorbehandelt, d. h., es wurde jeweils eine geringe Menge (4 x 200 ml) über den Filter gegeben.

1.3 Entfernung der Scrapie-Erreger

10 ml der in Beispiel 1 gewonnenen Scrapie-Spike-Lösung wurden 600 ml des Thymushomogenisats zugesetzt und bei 4 °C über Nacht stehen gelassen. Dann wurde die Lösung über einen ersten Vorfilter mit einer Porengröße von 2,0 µm, einen zweiten Vorfilter mit einer Porengröße von 0,8 µm und einen dritten Vorfilter mit einer Porengröße von 0,2 µm filtriert. Das so erhaltene Filtrat wurde als Probe A bezeichnet.

190 ml des gespikten Vorfiltrat-Materials wurden nochmals mit 6 ml der Scrapie-Spike-Lösung beaufschlagt und dann einer Ultrafiltration über eine Amiconmembran S1Y30 unterworfen. Das so erhaltene Filtrat wurde als Probe B bezeichnet.

144 ml von Probe B wurden nochmals mit 6 ml des Scrapie-Spike-Materials beaufschlagt und einer zweiten Ultrafiltration über eine weitere Amicon S1Y30-Membran unterworfen. Das hierdurch erhaltene Filtrat wurde als Probe C bezeichnet.

121 ml der Probe C wurden nochmals mit 6 ml des Scrapie-Spike-Materials versetzt und nochmals wie zuvor einer Ultrafiltration über die gleiche Membran unterworfen. Die hierbei erhaltene Lösung wird als Probe D erhalten.

121 ml der Probe D wurden einer vierten Ultrafiltration über die gleiche Membran unterworfen. Die hierbei erhaltene Lösung wurde als Probe E bezeichnet.

93 ml der Probe E wurden einer fünften Ultrafiltration mit der gleichen Membran unterworfen. Das hierbei erhaltene Filtrat wird als Probe F bezeichnet.

Die Probe F wurde anschließend nochmals durch eine Sterilfiltration über einen Filter von 0,2 $\mu$m geleitet. Das hierbei erhaltene Filtrat wird als Probe G bezeichnet.

Zum Überblick über das verwendete Abreicherungsverfahren wird auf Fig. 1 verwiesen.

1.4 Titration des infektiösen Erregers am Hamstermodell

Jeweils 12 Hamstern wurden 50 $\mu$l der unverdünnten bzw. logaritmisch verdünnten Proben A, B, C, D und G intracerebral injiziert. Für die Proben E und F wurden jeweils 24 Hamster mit nur 50 $\mu$l der unverdünnten Lösung intracerebral infiziert. Zur Bestimmung der Infektiosität des Scrapieagens wurden insgesamt 96 Hamster mit 50 $\mu$l des Spike-Materials mit verschiedenen Verdünnungen behandelt, wobei für jede Verdünnung 12 Hamster infiziert wurden.

50 Tage nach Injektion wurde der Zustand der Tiere bezüglich klinischer neurologischer Symptome beobachtet, und alle befallenen Tiere wurden in dem Terminalstadium der Erkrankung getötet und analysiert. Tiere, die keine Symptome zeigten, wurden nach Beendigung der Studie, d.h. ca. 450 Tage nach Injektion, ebenfalls getötet und untersucht.

[0036] - Titration des Scrapie-Spike-Materials (positive Kontrolle) Nach Beendigung der Studie zeigten alle Hamster, die mit dem unverdünnten Spike-Material sowie mit Verdünnungen von $10^{-1}$ bis $10^{-5}$ infiziert worden waren, eine histopathologisch bestätigte Scrapie-Infektion. Bei den Verdünnungen von $10^{-6}$ und $10^{-7}$ zeigten 7 von 12 Hamstern eine Infektion, die ebenfalls durch histopathologische Untersuchungen bestätigt wurde. Keiner der Hamster, die mit der $10^{-8}$ verdünnten Lösung infiziert worden ist, zeigte irgendwelche klinischen oder histopathologischen Symptome der Scrapie-Erkrankung.

Probe A (nach Vorfiltration)

Hamster, die mit der unverdünnten Probe A und mit Verdünnungen bis zu $10^{-5}$ infiziert wurden, zeigten das Auftreten von Scrapie. Bei keinem der Hamster, die mit den $10^{-6}$- und $10^{-7}$-fachen Verdünnungen infiziert wurden, konnte der Ausbruch der Erkrankung beobachtet werden.

Probe B (Produkte der ersten Ultrafiltration)

Hier wurden nur noch nach Injektion mit der unverdünnten sowie mit den $10^{-1}$- und $10^{-2}$-fachen Verdünnungen das Auftreten von Scrapie-Infektionen beobachtet. Bei weiteren Verdünnungen konnte kein Auftreten der Erkrankung mehr beobachtet werden.

Proben C bis G

Nach Beendigen der Studie wurde mit diesen Produkten kein Auftreten einer Scrapie-Infektion in den Proben C, E, F und G gefunden. Lediglich 2 Hamster, die mit der unverdünnten Probe D infiziert wurden, entwickelten klinische Anzeichen einer Scrapie-Infektion. In diesem Zusammenhang wird nochmal darauf hingewiesen, daß die Probe D nach dem Respiking des Filtrates mit Scrapie-Homogenisat erhalten wurde.

Die Ergebnisse der Hamster-Inokulation und die Ergebnisse der Histopathologie und des Infektionstiters (LD50/ml) sind in Tabelle 1 angegeben. Dabei wurde der Infektionstiter unter Verwendung der Methode von Karber (1931, Beitrag zur kollektiven Behandlung pharmakologischer Reinversuche; Archives of experimental pathology and pharmacology, 162, 480 - 483) wie folgt bestimmt:

$$m = X + 1/2 \, d - d \, \frac{(\Sigma rl)}{(n)}$$

wobei

m   der durchschnittliche LD50-Wert pro Inokulationsvolumen bedeutet,
X   der Reziprokwert der höchsten infektiösen Verdünnung bedeutet,
d   der Logarithmus des Verdünnungsintervalls,
rl   die Anzahl der bei jeder Verdünnung nicht infizierten Tiere und
n   die Anzahl der mit jeder Verdünnung inokulierten Tiere

bedeutet.

Auf diese Weise ergab sich für das Ausgangsmaterial ein Infektionstiter von $10^{8,45}$ LD50/ml. Nach drei Vorfiltrationen für die Probe A $10^{4,97}$ LD50/ml betrug die Gesamtzahl der infektiösen Erreger vor der ersten Ultrafiltration somit nach dem Respiken (6 ml x $10^{8,45}$) $10^{9,23}$ LD50-Einheiten (150 ml). Nach Ultrafiltration enthielt die Probe B nur noch $10^{4,56}$ LD50-Einheiten ($10^{2,38}$ LD50/ml). Dies ergibt eine Abreicherung des Erregers bei der ersten Ultrafiltration um den Faktor $10^{4,67}$.

Abreicherung bei der zweiten Ultrafiltration

Nach dem Respiken der Probe (6 ml x $10^{8,45}$) enthielt die Lösung (127 ml) insgesamt $10^{9,23}$ LD50 Erreger. Der trotz erneuter Beaufschlagung gefundene Infektionstiter nach der zweiten Ultrafiltration war kleiner als 1 LD50/ml, d. h. es wurde kein Erreger mehr gefunden. Dies ergibt einen Titer in 127 ml von < $10^2$ LD50. Damit ist die Abreicherung im zweiten Ultrafiltrationsschritt größer als $10^{7,13}$.

Wie zuvor beschrieben wurde die Probe G bestimmt. Hier wurde eine Abreicherung um einen Faktor von > $10^{7,28}$ gefunden.

Fig. 2 zeigt die Abreicherungsraten des Scrapie-Erregers durch serielle Ultrafiltration. Entgegen dem theoretisch zu erwartenden Ergebnis einer konstanten oder sogar abnehmenden Abreicherung, wie sie etwa bei der seriellen Filtration des Bakteriophagen fr über die gleiche Ultrafiltrationsmembran S1Y30 beobachtet wird (WO 91/12027, Tabelle 3), nimmt die Abreicherungsrate für den Scrapie-Erreger nach dem ersten Filtrationsschritt mehr als 2 Zehnerpotenzen zu.

Durch die serielle Ultrafiltration kann somit selbst die Titerreduktion durch die als sicher geltenden Autoklavier- oder Alkalibehandlung um ein Erhebliches unterschritten und damit ein Arzneimittel ohne Gefährdungspotential durch Erreger der spongioformen Enzephalopathien hergestellt werden. Unabhängig davon können natürlich zusätzliche Vor- oder Nachreinigungsschritte mit dem erfindungsgemäßen Verfahren kombiniert werden. Das erfindungsgemäße Verfahren zeichnet sich dadurch aus, daß die Erreger durch Ultrafiltration entfernt werden, bis das Produkt mit Sicherheit nicht mehr infektiös wirkt.

Die vorliegenden Versuche belegen, daß mit einer wiederholten Ultrafiltration die Entfernung von infektiösen Erregern, wie Prionen bzw. sogenannten "langsamen Viren" die Abreicherungsgeschwindigkeit pro Filtrationsschritt um mindestens das 100- bis 1000-fache ansteigt, was zu einer rapiden Beschleunigung der Entfernung des Erregers und damit auch zu einer Infektionssicherheit des so erhaltenen Produktes führt.

TABELLE 1

Entfernen eines Rodent Adaptierten Scrapie Agens

| Probe | Log. Verdünnung | behandelte Tiere | zwischenzeitlich aufgetretene Todesfälle[1] | Tiere mit Scrapie[2] | durchschnittliche Inkubationsdauer (SE)[3] | Titer der Infektivität (LD 50/ml)[+] |
|---|---|---|---|---|---|---|
| A | unverdünnt | 12 | 0 | 12 | 93.17 (3.71) | |
| | $10^{-1}$ | 12 | 0 | 12 | 120.75 (13.90) | |
| | $10^{-2}$ | 12 | 0 | 12 | 105.58 (2.01) | |
| | $10^{-3}$ | 12 | 0 | 8 | 132.13 (15.28) | $10^{4.97}$ |
| | $10^{-4}$ | 12 | 0 | 5 | 197.40 (42.37) | |
| | $10^{-5}$ | 12 | 1 | 1 | 112.00 | |
| | $10^{-6}$ | 12 | 0 | 0 | - | |
| | $10^{-7}$ | 12 | 0 | 0 | - | |
| B | unverdünnt | 12 | 1 | 11[8] | 124.09 (13.22) | |
| | $10^{-1}$ | 12 | 1 | 5 | 168.00 (20.65) | |
| | $10^{-2}$ | 12 | 0 | 2 | 126.00 (0.00) | |
| | $10^{-3}$ | 12 | 1 | 0 | - | $10^{2.38}$ |
| | $10^{-4}$ | 12 | 0 | 0 | - | |
| | $10^{-5}$ | 12 | 0 | 0 | - | |
| | $10^{-6}$ | 12 | 0 | 0 | - | |
| | $10^{-7}$ | 12 | 1 | 0 | - | |

EP 0 776 212 B1

TABELLE 1 - Fortsetzung

Entfernen eines Rodent Adaptierten Scrapie Agens

| Probe | Log. Verdünnung | behandelte Tiere | zwischenzeitlich aufgetretene Todesfälle [1] | Tiere mit Scrapie [2] | durchschnittliche Inkubationsdauer (SE) [3] | Titer der Infektivität (LD 50/ml) [4] |
|---|---|---|---|---|---|---|
| C | unverdünnt | 12 | 1 | 0 | - | |
| | $10^{-1}$ | 12 | 0 | 0 | - | |
| | $10^{-2}$ | 12 | 0 | 0 | - | |
| | $10^{-3}$ | 12 | 0 | 0 | - | 0 |
| | $10^{-4}$ | 12 | 1 | 0 | - | |
| | $10^{-5}$ | 12 | 0 | 0 | - | |
| | $10^{-6}$ | 12 | 1 | 0 | - | |
| | $10^{-7}$ | 12 | 0 | 0 | - | |
| D | unverdünnt | 12 | 0 | 2 | 146.00 (14.00) | |
| | $10^{-1}$ | 12 | 0 | 0 | - | |
| | $10^{-2}$ | 12 | 1 | 0 | - | |
| | $10^{-3}$ | 12 | 0 | 0 | - | $\leq 10^{7.03}$ |
| | $10^{-4}$ | 12 | 0 | 0 | - | (s. Fuß- |
| | $10^{-5}$ | 12 | 0 | 0 | - | note 7) |
| | $10^{-6}$ | 12 | 1 | 0 | - | |
| | $10^{-7}$ | 12 | 1 | 0 | - | |

TABELLE 1 - Fortsetzung

Entfernen eines Rodent Adaptierten Scrapie Agens

| Probe | Log. Verdünnung | behandelte Tiere | zwischenzeitlich aufgetretene Todesfälle[4] | Tiere mit Scrapie[2] | durchschnittliche Inkubationsdauer (SE)[3] | Titer der Infektivität (LD 50/ml)[4] |
|---|---|---|---|---|---|---|
| E | unverdünnt | 12 | 0 | 0 | - | 0 |
| F | unverdünnt | 12 | 1 | 0 | - | 0 |
| G | unverdünnt | 12 | 0 | 0 | - | |
| | $10^{-1}$ | 12 | 0 | 0 | - | |
| | $10^{-2}$ | 12 | 0 | 0 | - | |
| | $10^{-3}$ | 12 | 0 | 0 | - | 0 |
| | $10^{-4}$ | 12 | 1 | 0 | - | |
| | $10^{-5}$ | 12 | 0 | 0 | - | |
| | $10^{-6}$ | 12 | 0 | 0 | - | |
| | $10^{-7}$ | 12 | 0 | 0 | - | |

EP 0 776 212 B1

TABELLE 1 - Fortsetzung

Entfernen eines Rodent Adaptierten Scrapie Agens

| Probe | Log. Verdünnung | behandelte Tiere | zwischenzeitlich aufgetretene Todesfälle[1] | Tiere mit Scrapie[2] | durchschnittliche Inkubationsdauer (SE)[3] | Titer der Infektivität (LD 50/ml)[4] |
|---|---|---|---|---|---|---|
| SPIKE MATERIAL[5] | $10^{-1}$ | 12 | 0 | 12 | 73.00 (1.77) | |
| | $10^{-2}$ | 12 | 0 | 12 | 75.42 (1.48) | |
| | $10^{-3}$ | 12 | 0 | 12 | 84.75 (1.91) | $10^{8.45}$ |
| | $10^{-4}$ | 12 | 0 | 12 | 91.00 (0.86) | |
| | $10^{-5}$ | 12 | 0 | 12 | 105.00 (3.11) | |
| | $10^{-6}$ | 12 | 0 | 7 | 153.00 (32.02) | |
| | $10^{-7}$ | 12 | 0 | 7 | 152.29 (23.60) | |
| | $10^{-8}$ | 12 | 1 | 0 | - | |

1 Todesfälle, die nicht mit dem Auftreten von Scrapie vor dem 343. Tag erfolgten - durch Histologie bestätigt

2 Durch Histologie bestätigt

3 (SE) = Standardabweichung

4 Titer der Infektivität, berechnet nach Karber

5 Beaufschlagung mit 30 % Gew./Vol. Homogenisat, die 1:3 verdünnt war

6 Korrigiert für die 1:3 Verdünnung des ursprünglichen 30 % Gew./Vol. Homogenisats

7 Aufgrund der niedrigen Anzahl bei einer Verdünnung infizierten Tiere wurde zur Berechnung des Titers eine Modifikation von Karber verwendet

8 Diese Gruppe wurde bei der Berechnung des Titers nach der Methode von Karber nicht verwendet.

**Patentansprüche**

1. Verfahren zur Herstellung von pharmazeutischen Präparaten und/oder Nahrungsmitteln aus möglicherweise mit Prionen verseuchtem Ausgangsmaterial, umfassend die sichere Entfernung der infektiösen Erreger, dadurch gekennzeichnet, daß die Prionen lediglich dadurch entfernt werden, daß man das zu reinigende Material wiederholt über eine Ultrafiltrationsmembran und/oder über eine Serie von Ultrafiltrationsmembranen filtriert.

2. Verfahren nach Anspruch 1, dadurch gekennzeichnet, daß das infektiöse Material ein Erreger der spongiformen Enzephalopathie ist.

3. Verfahren nach einem der vorhergehenden Ansprüche, dadurch gekennzeichnet, daß der Erreger Scrapie, BSE, Kuru, Gerstmann-Sträussler, Jakob-Creutzfeldt und/oder Alzheimer verursacht.

4. Verfahren nach einem der vorhergehenden Ansprüche, dadurch gekennzeichnet, daß man die Filtrationsmembran vor ihrer Verwendung mit dem zu filtrierenden Ausgangsmaterial vorbehandelt.

5. Verfahren nach einem der vorhergehenden Ansprüche, dadurch gekennzeichnet, daß man ab der zweiten Ultrafiltration den Erreger um mindestens einen Faktor von $10^{-6}$, vorzugsweise mindestens $10^{-7}$, abreichert.

6. Verfahren nach einem der vorhergehenden Ansprüche, dadurch gekennzeichnet, daß das pharmazeutische Präparat Thymusfraktionen, Heparin, natürliche und/oder rekombinante Proteine aus Zellkulturen und/oder humanen menschlichen Wachstumsfaktor enthält.

7. Verfahren nach einem der vorhergehenden Ansprüche, dadurch gekennzeichnet, daß das pharmazeutische Präparat Interleukin-2, rekombinantes Glykoprotein und/oder Gewebs-Plasminogen-Aktivator umfaßt.

8. Verfahren nach einem der vorhergehenden Ansprüche, dadurch gekennzeichnet, daß man als Ultrafiltrationsmembran eine Amicon Spiralmembran mit Tangentialfluß S1Y30, S10Y30 oder S100Y30 verwendet.

9. Verfahren nach einem der vorhergehenden Ansprüche, dadurch gekennzeichnet, daß man die Ultrafiltrationsmembran durch Beaufschlagen mit Viren der Familie Leviviridae validiert.

10. Verfahren nach einem der vorhergehenden Ansprüche, dadurch gekennzeichnet, daß es mittels einem Druckhaltetest kontrolliert wird.

**Claims**

1. Method of producing pharmaceutical preparations and/or foodstuffs from raw material which has possibly been contaminated with prions, including the safe removal of the infectious pathogens, characterized in that the prions are only removed when the material to be purified is repeatedly filtered via an ultrafiltration membrane and/or via a series of ultrafiltration membranes.

2. Method according to claim 1, characterised in that the infectious material is a pathogen of the spongiform encephalopathy.

3. Method according to one of the preceding claims, characterised in that the pathogen causes scrapie, BSE, kuru, Gerstmann Sträussler's disease, Jakob Creutzfeldt disease and/or alzheimer's disease.

4. Method according to one of the preceding claims, characterised in that, before being used, the filtration membrane is pretreated with the raw material to be filtered.

5. Method according to one of the preceding claims, characterised in that the pathogen is depleted by at least a factor of $10^{-6}$, preferably at least $10^{-7}$, from the second ultrafiltration onwards.

6. Method according to one of the preceding claims, characterised in that the pharmaceutical preparation contains thymus fractions, heparin, natural and/or recombinant proteins of cell cultures and/or humane human growth factor.

7. Method according to one of the preceding claims, characterised in that the pharmaceutical preparation includes interleucine-2, recombinant glycoprotein and/or tissue plasminogen activator.

8. Method according to one of the preceding claims, characterised in that an amicon spiral membrane having tangential flow S1Y30, S10Y30 or S100Y30 is used as the ultrafiltration membrane.

9. Method according to one of the preceding claims, characterised in that the ultrafiltration membrane is validated by the introduction of viruses of the leviviridae family.

10. Method according to one of the preceding claims, characterised in that it is controlled by means of a pressure-maintaining test.

**Revendications**

1. Procédé d'obtention de préparations pharmaceutiques ou de produits alimentaires à partir de substances de départ pouvant être infectées par des prions, comprenant l'élimination certaine des agents infectueux, caractérisé en ce que les prions sont éliminés seulement par le fait qu'on filtre plusieurs fois la substance à purifier sur une membrane d'ultrafiltration ou une série de membranes d'ultrafiltration.

2. Procédé selon la revendication 1 caractérisé en ce que, la substance infectueuse provoque l'encéphalopathie spongiforme.

3. Procédé selon n'importe laquelle des revendications précédentes, caractérisé en ce que l'agent pathogène provoque la tremblante du mouton, l'ESB, le kuru, les maladies de Gerstmann-Straussler, de Jacob-Creuzfeldt et/ou d'Alhzheimer.

4. Procédé selon n'importe laquelle des revendications précédentes, caractérisé en ce que la membrane de filtration est traitée avant l'application de la substance de départ à filtrer.

5. Procédé selon n'importe laquelle des revendications précédentes, caractérisé en ce que à la deuxième ultrafiltration l'agent pathogène est réduit d'un facteur d'au moins $10^{-6}$, de préférence d'au moins $10^{-7}$.

6. Procédé selon n'importe laquelle des revendications précédentes, caractérisé en ce que la préparation pharmaceutique comprend un extrait du thymus, ou contient de l'héparine, des protéines naturelles ou recombinantes de cultures de cellules et/ou le facteur de croissance humaine.

7. Procédé selon n'importe laquelle des revendications précédentes, caractérisé en ce que la préparation pharmaceutique contient de l'interleukine-2, une glycoprotéine recombinante et/ou un activateur tissulaire de plasminogène.

8. Procédé selon n'importe laquelle des revendications précédentes, caractérisé en ce que comme membrane d'ultrafiltration on utilise une membrane en spirale Amicon avec flux tangentiel S1Y30, S10Y30 ou S100Y30.

9. Procédé selon n'importe laquelle des revendications précédentes, caractérisé en ce que la membrane d'ultrafiltration est validée par traitement avec des virus de la famille des Leviviridae.

10. Procédé selon n'importe laquelle des revendications précédentes, caractérisé en ce qu'il est contrôlé par un test de maintien à la pression.

Abreicherung des Scrapie-Erregers durch Ultrafiltration

```
                    Thymusdrüse vom Rind
                          |
                          |   Homogenisieren
                          v
Beaufschlagen mit
SCRAPIE       ---------> |
                          |
                          v
                     AUTODIGESTION                          ABREICHERUNG
                       20 HR, 4°C
                          |
                          v
              FILTRATION, NYLON   GAZE
                          |
                          v
                   PREFILTRATION
                   FILTER 1 (2.0 um)
                   FILTER 2 (0.8 um)
                   FILTER 3 (0.2 um)                            $10^{1.23}$
                          |
                          v
Einstellen der Proteinkonzentration und der Salzkonzentration
                     |------> Probe A (Stufe 6)
  Beaufschlagen mit|
  SCRAPIE    ----->|
                     v
                   ULTRAFILTER 1                               $10^{4.67}$
                     |
                     |------> Probe B (Stufe 9)
  Beaufschlagen mit|
  SCRAPIE    ----->|
                     v
                   ULTRAFILTER 2                              $> 10^{7.13}$
                     |
  Beaufschlagen mit|------> Probe C (Stufe 13)
  SCRAPIE    ----->|
                     v
                   ULTRAFILTER 3                              $> 10^{7.28}$
                     |
                     |------> Probe D (Stufe 17)
                     |
                     v
                   ULTRAFILTER 4
                     |
                     |------> Probe E (Stufe 19)
                     |
                     v
                   ULTRAFILTER 5
                     |
                     |------> Probe F (Stufe 21)
                     |
                     v
               0.2 um FILTER
                     |
                     |------> Probe G (Stufe 23)
```

FIG. 1

FIG. 2    Abreicherung des Erregers von Scrapie durch mehrfache Ultrafiltration

EP 0 776 212 B1